(19) Europäisches Patentamt
European Patent Office
Office européen des brevets

(11) Publication number : **0 471 561 A2**

# (12) EUROPEAN PATENT APPLICATION

(21) Application number : **91307493.6**

(22) Date of filing : **14.08.91**

(51) Int. Cl.$^5$ : **B01J 31/18,** C07C 29/50, C07B 33/00

(30) Priority : **16.08.90 US 568116**

(43) Date of publication of application : **19.02.92 Bulletin 92/08**

(84) Designated Contracting States : **BE DE FR GB IT NL**

(71) Applicant : **Sun Refining and Marketing Company
1801 Market Street
Philadelphia, PA 19103-1699 (US)**

(72) Inventor : **Ellis Jr. Paul E.
1179 Glenside Avenue,
Downingtown, PA 19335 (US)**
Inventor : **Lyons, James E.
211 Cooper Drive,
Wallingford, PA 19086 (US)**
Inventor : **Myers Jr. Harry K.
Limestone Road,
Cochranville, PA 19330 (US)**

(74) Representative : **Lewin, John Harvey
Elkington and Fife Prospect House 8
Pembroke Road
Sevenoaks, Kent TN13 1XR (GB)**

## (54) Metal coordination complex catalysts containing a halogenated ligand for hydrocarbon oxidation.

(57) Perhalogenated metal porphyrins and metal phthalocyanines, wherein the metal is iron, chromium, manganese, ruthenium and/or copper, are useful as oxidation catalysts for oxidation of aliphatic hydrocarbons with air or oxygen.

EP 0 471 561 A2

## BACKGROUND OF THE INVENTION

This application is related to U.S. Serial No. 466,163 filed January 17, 1990; U.S. Serial No. 66,666 filed June 20, 1987, now U.S. Patent 4,900,871; U.S. Serial No. 246 filed January 2, 1987, now U.S. Patent 4,895,682; and U.S. Serial No. 247 filed January 2, 1987, now U.S. Patent 4,895,680.

This invention relates to novel catalysts for the oxidation of hydrocarbons. More particularly, this invention relates to catalysts for oxidizing a wide range of oxidizable hydrocarbons, particularly alkanes, with air or oxygen. The catalyst is, for example, an azide, hydroxide, halide or nitride of a halogenated porphyrin or phthalocyanine complex of metals such as iron.

The oxidation of alkanes and other aliphatic hydrocarbons catalyzed by transition metal complexes in the liquid phase is well known in the art, and commercial applications of this technology are extensive. See, for example, J. E. Lyons, Hydrocarbon Processing, November, 1980, 107, Table I.

However, the selective partial oxidation of unactivated hydrocarbons such as methane, ethane, propane, butanes and the like by air or oxygen as the oxidant is extremely difficult to achieve. The use of macrocyclic metal complexes such as metalloporphyrins as catalyst in such oxidations has not provided high reaction rates and selectivities under mild conditions. Some success has been achieved using two less economically desirable approaches:

1) The use of metalloporphyrin catalysts such as Fe(TPP)Cl and Mn(TPP)Cl (where "TPP" is the dianion of 5,10,15,20-tetraphenylporphine) with iodosylbenzene, sodium hypochlorite, alkylhydroperoides or other expensive non-regenerable oxidants. [P. Traylor, D. Dolphin, and T. Traylor, J. Chem. Soc. Chem. Comm., 279 (1984); J. Groves, W. Kruper, Jr., and R. Haushalter, J. Am. Chem. Soc., 102, 6377 (1980); C. Hill, and B. Schardt, J. Am. Chem. Soc., 102, 6374 (1980); J. Smegal and C. Hill, J. Am. Chem. Soc., 105, 3515 (1983); A. Middleton and D. Smith, U.S. Patent 4,459,427 (July 10, 1984)]; and

2) The use of metalloporphyrin catalysts with molecular oxygen as oxidant and simultaneous addition of a reductant such as $NaBH_4$, ascorbic acid or colloidal platinum with $H_2$. Again, the added reagents are expensive and non-regenerable. Examples of this approach can be found in D. Mansuy, M. Fontecave, and J. Bartoli, J. Chem. Soc. Chem. Comm. 253 (1983); I. Tabushi and A. Yazaki, J. Am. Chem. Soc., 103, 7371 (1981).

U.S. Patent 4,892,941 discloses halogenated tetraphenyl porphyrins which are said to be useful for the oxidation of alkanes. Also, certain halogenated phthalocyanines are described in A Fluorinated Iron Phthalocyanine, J. G. Jones, et al, Inorganic Chemistry, Vol. 8, page 2018 (1969)

Our '680 and '682 patents described above disclose the use of azide and nitride transition metal ligand, i.e., coordination complexes, for the air oxidation of alkanes and other hydrocarbons. Our '871 patent describes the use of iron halogenated coordination complexes for similar oxidations. It is disclosed therein that halogenating the coordination complex portion of the catalyst greatly increases the activity of the catalyst. While it was known to fluorinate iron and manganese tetraphenylporphyrinato chloride catalysts to improve their stability in the oxidation of alkanes and alkenes using strong oxidizers such as iodosylbenzene (C. Chang and F. Ebina, J. Chem. Soc.Chem. Comm., 778 (1981)), it was not known for the oxidation of alkanes and alkenes with air or oxygen. However, we have now additionally and unexpectedly found greatly increased catalyst activity with the metal porphyrin and phthalocyanine coordination complexes when the ligand is perhalogenated, with the iron species of our catalysts being even better (in the oxidation to alcohols) than the rest.

This invention relates to the improved halogenated metal coordination complex catalysts described above for the oxidation of hydrocarbons, and particularly alkanes, using air or oxygen, and without the need for added expensive, non-regenerable oxidants, reductants, or other co-catalysts.

## SUMMARY OF THE INVENTION

In accordance with the present invention, it has now been found that catalysts comprising a perhalogenated porphyrin or phthalocyanine coordination complex containing one or more metal cations, as defined below, such as iron tetrakis(pentafluorophenyl)octabromoporphyrinatoazide are particularly effective for the oxidation of alkanes having from about 1 to 20 carbon atoms, and preferably those having from 1 to 10 carbons atoms, more preferably 1 to 4, such as methane, ethane, propane, and butanes. More particularly, it has been found that perhalogenating the ligand systems of these metal-containing coordination complexes greatly improves the activity of these catalysts for alkane oxidations.

The use of these catalysts in the oxidation of hydrocarbons, and especially alkanes, results in several unexpected advantages. As in our issued patents described above, the reaction can be carried out at lower temperatures than heretofore employed; there is often little or no cleavage of the starting material; there is little or no formation of CO or $CO_2$; there is higher selectivity for alcohols, when alcohols are the desired product; the reaction rates are generally faster than those of comparable prior art processes; and the processes themselves are less expensive than those of the prior art which require non-$0_2$ oxidants. However, in addition, the specific use of the per-halogenated ligand in the catalyst coordination complex results in surprisingly higher catalyst activity and, with iron as the metal, even higher selectivity for alcohols, when alcohols are the desired product, than are obtained with partially or unhalogenated metal coordination complexes.

## DESCRIPTION OF THE INVENTION

As stated, our catalysts are particularly effective in the oxidation of alkanes, and alkenes including cycloalkanes, substituted alkanes and alkenes and the like. The starting materials thus include straight and branched-chain compounds having from about 1 to 20 carbon atoms, preferably 1 to 10 carbon atoms, such as methane, ethane, propane, n-butane, isobutane, n-pentane, n-hexane, 2-methylpentane, 3-methylpentane, heptane, 2-methylheptane, 3-methylheptane the corresponding alkene forms, and the like, as well as cycloalkanes and alkenes having from about 5 to 20 carbon atoms, preferably 5 to 10 carbon atoms, such as cyclopentane, cyclohexane, cycloheptane, cyclooctane, the corresponding alkene forms, and the like. These compounds, if desired, may be substituted with various moieties, although care should be taken to exclude substituents which will adversely affect the activity of the catalyst.

The oxidation, which nay be carried out in a generally known manner, is desirably conducted in the liquid phase, although this is not critical, using such organic solvents as benzene, acetic acid, acetonitrile, methyl acetate, or like solvents which are inert to the conditions of the reactions, or in a neat solution of the hydrocarbon if it is liquid, and under pressures ranging from about 15 to 1500 psig, preferably 30 to 750 psig, at temperature of from about 25 to 250°C, more preferably 70 to 180°C. Depending upon whether the hydrocarbon to be oxidized is a solid, liquid, or gas, it is dissolved in or bubbled through the solvent, together with air or oxygen, in the presence of the aforementioned iron coordination complex catalyst for periods of time sufficient to yield the desired oxidation product, generally from about 0.5 to 100 hours, and more preferably from 1 to 10 hours. "Iron" as used herein includes both the Fe(II) and Fe(III) states.

The choice of solvent, while not critical, can have an effect on the rates and selectivities obtained and should be selected carefully in order to optimize the desired results. For example, it has been found that solvents such as acetonitrile and acetic acid are often very effective for the oxidation of alkanes to form oxygen-containing compounds, whereas reactions carried out in solvents such as methyl acetate or benzene may occur more slowly. Thus, by routine experimentation the optimum solvent for the particular process can readily be determined.

The ratios of the various reactants may vary widely, and are not critical. For example. the amount of catalyst employed can range from about $10^{-6}$ to $10^{-3}$ moles per mole of hydrocarbon such as alkane, and more preferably from about $10^{-5}$ to $10^{-4}$ mole of catalyst per mole of hydrocarbon, although other amounts are not precluded; while the amount of oxygen relative to the hydrocarbon starting material may also vary widely, generally $10^{-2}$ to $10^{2}$ moles of oxygen per mole of hydrocarbon. Care should be taken since some of the ratios fall within explosive limits. As a group, the catalysts are almost always soluble unless used in large excess. Thus, as a rule the reactions are generally carried out homogeneously.

The catalysts of our invention may best be defined as metal coordination complexes having the following general structure:

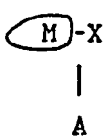

wherein M is iron, manganese, chromium ruthenium, or copper, or combinations thereof, preferably one of the first four mentioned, more preferably the first three, more preferably iron. The component depicted as "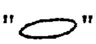" is a porphyrin or phthalocyanine ligand as described below which additionally contains a halogen moiety, X. A is an anion such as oxo, nitride ($N^{-3}$), hydroxide (OH), azide ($N_3$), halide (F, Cl, Br, I) or the like. "A" can also be absent in the case of porphyrin ligands if the metal component is in reduced form, e.g., Fe(II) instead of Fe(III).

Of the metals described, iron is preferred because it exhibits higher activity than the others. On the other hand, other metals have their own advantages; manganese is more selective to alcohols and chromium is more selective to ketones. Thus, the choice of metal will depend on the user's particular needs.

The iron porphyrin or phthalocyanine nitride catalysts also exist as "dimers" such as the μ-nitrido on μ-oxo dimers, $(FeTPPX_{28})_2R$ and $(FePcX_{16})_2R$, where R = "N" or "O", which are included within the catalysts of our invention.

The term "ligand" is used herein in its conventional meaning and refers generically to a group or system of atoms which forms one or more bonds to a metal ion, i.e., forms a coordination complex, and stabilizes this metal coordination complex in desirable oxidation states. Suitable ligands for the present purpose are the well-known phthalocyanines and porphyrins (sometimes referred to as porphyrinatos) such as alkyl and aryl porphyrins such as tetraphenylporphyrins, octaethylporphyrins, tetramethylporphyrins and the like. Usually there are 0-12 substituents, alkyl or aryl, on the basic porphyrin structure, the alkyls are $C_1$-$C_4$ and the aryls contain 1 or 2 rings which may themselves have alkyl substituents.

The halogen component of the ligand, X, can be fluoride, chloride, bromide, iodide or mixtures thereof, but preferably is one of the first three mentioned, more preferably fluoride. The degree of ligand halogenation should be complete, i.e., at least 90%, preferably 100%, which is customarily referred to as perhalogenation for which the conventional symbols are F-, Cl-, etc. We have found that complete halogenation provides substantially superior results.

The catalysts of our invention can be readily prepared by simple modifications of procedures described in the art for preparing unhalogenated ligands. For example, the unhalogenated Fe(TPP)Cl complex (in which "TPP" is tetraphenylporphyrinato) can be prepared by a standard method in which $(TPP)H_2$ and Fe(II) (or other metal) chloride are refluxed together in a dimethylformamide solution. Purification is achieved by chromatography. (See, e.g., A. D. Adler et al, J. Inorg. Nucl. Chem., 32, 2443 (1970).) From these metal salts the corresponding azides may be prepared by metathesis reactions with dissolved $NaN_3$ or hydrazoic acid.

To prepare the corresponding halogenated ligand coordination complex of this invention, one or more of the precursors of the ligand are halogenated before the ligand itself is produced by a condensation reaction. Thus, fluorination of benzaldehyde followed by condensation with pyrrole yields $TPPF_{20}$ in which $F_{20}$ refers to twenty fluorine atoms on the four phenyls. Substituting this $TPPF_{20}$ for TPP in the aforementioned method of refluxing in a dimethylformamide solution containing the Fe(II) will yield the corresponding $Fe(TPPF_{20})$ salt.

By way of specific illustration the perhalogenated metal porphyrin, $[Fe(TPPF_{20}Br_8)]Cl$, iron (tetrakispentafluorophenyloctabromoporphyrin) chloride is prepared as follows: Under $N_2$, a flask is charged with 1.0 g of $Zn(TPPF_{20})$ and 300 ml of $CCl_4$. This mixture is refluxed with 150 ml of 6M $Br_2$ for 5 hours and is then allowed to cool to room temperature. After chromatograpy on basic alumina, 300 mg. of pure $Zn(TPPF_{20}Br_8)$ is obtained and characterized by UV/VIS, IR and elemental analysis. The zinc is removed by acid treatment and the iron complex $Fe(TPPF_{20}Br_8)$ Cl is prepared by $FeCl_2$ treatment in refluxing DMF. The azide, $Fe(TPPF_{20}Br_8)N_3$, can be prepared by reaction of the chloride salt with $NaN_3$ in acetone. The ruthenium, chromium and manganese complexes are prepared similarly. The hydroxo salt, $Fe(TPPF_{20}Br_8)OH$, is prepared from the chloro salt by treatment with dilute aqueous KOH in $CH_2Cl_2/H_20$.

The perhalogenated metal porphyrin $Fe(TPPF_{20}Cl_8)Cl$ is prepared as follows: under $N_2$, 0.5 g of $Zn(TPPF_{20})$ dissolved in 500 ml of $CCl_4$ is refluxed for 5 hr. while $Cl_2$ gas is bubbled slowly through the solution. After cooling the mixture is filtered and chromatographed on alumina, yielding 0.4 g of pure Zn $(TPPF_{20}Cl_8)$. The zinc is removed by trifluoroacetic acid treatment, and the iron is then inserted by reaction with $FeCl_2$ in DMF. The resulting $Fe(TPPF_{20}Cl_8)Cl$ is characterized by UV/VIS, IR, and elemental analysis. The ruthenium, manganese, and chromium complexes are prepared similarly. The azide salts are prepared from the chloride salts by metathesis with $NaN_3$ in acetone. The hydroxo salt, $Fe(TPPF_{20}Cl_8)OH$, is prepared from the chloro salt by treatment with dilute aqueous KOH solution in $CH_2Cl_2$.

The perfluorinated metal porphyrin, iron perfluorotetraphenylporphyrin chloride, $Fe(TPPF_{28})Cl$ (28 F atoms) can be prepared by the reaction of dilute $F_2$ gas in $N_2$ with $Zn(TPPF_{20})$ in $CCl_4$, with small added amounts of $CoF_3$, followed by removal of zinc and incorporation of iron as before. This porphyrin complex is analyzed by IR, UV/VIS, and elemental analysis. The ruthenium, chromium, and manganese complexes are prepared in analagous fashion. The azide salts are prepared from the chloride salts by reaction with $NaN_3$ in acetone. The hydroxo salt, Fe $(TPPF_{28})OH$ is prepared by the dilute aqueous KOH treatment of the chloro salt in $CH_2Cl_2$.

The preparation of the following iron complexes are examples of the tetraalkylporphyrins used in our invention. Freshly distilled pyrrole (0.8g) and trifluoroacetaldehyde methyl hemiacetal (10.9g) are refluxed for 24 hr. in 500 ml of ethanol containing 10 ml of 48% HBr. After neutralization of the mixture and extraction of the crude tetrakis(trifluoromethyl) porphyrin into $CH_2Cl_2$, the $H_2(TTFMP)$ is purified by chromatography with alumina. Iron is inserted into the $H_2(TTFMP)$ by $FeCl_2/DMF$ treatment giving Fe(TTFMP)Cl. The azide and hydroxide complexes are prepared by metathesis with $NaN_3$ in acetone and aqueous KOH in $CH_2Cl_2$, respectively. The pyrrolic hydrogens of this porphyrin can be partially or fully halogenated with Br, Cl, or F using the same techniques used for the tetraphenylporphyrins. As an example, dilute $F_2$ gas treatment of Zn(TTFMP) in the presence of $CoF_3$ in $CCl_4$ leads to isolation of the perfluorinated zinc porphyrin, zinc perfluorotetramethylporphyrin Zn-

(FTMP). Removal of the zinc by strong acid treatment leads to the metal free $H_2$(FTMP) from which the iron complex Fe(FTMP)Cl can be prepared by $FeCl_2$/DMF treatment. The azide, hydroxide, and nitride complexes are prepared in similar fashion to those described before. The chromium, manganese, and ruthenium complexes can be prepared from $H_2$FTMP by use of the appropriate metal chloride or metal acetate in DMF.

Other metal halogenate porphyrins or phthalocyanines are made analogously to the above methods. Similarly, when other porphyrin compounds are used similar results are obtained. The excellent catalytic activity of our catalyst depends on the electronic and structural nature of the porphyrin and phthalocyanine macro structure itself, not on any specific substituted group.

From the foregoing it will be seen that our novel catalysts are comprised of the component parts: the ligand moiety, which has beer completely halogenated, the metal center which is bound to (i.e., complexed with) the ligand, and as anion, azide, chloride, hydroxide or nitride or the like, which is bound to the metal. The metal-ligand portion is also frequently described in the art as a metal coordination complex. As noted, in some cases, the "dimers" are suitable catalysts and should be regarded as the equivalent thereof. In these "dimers", or, more accurately, dimetal compounds, each of the two iron centers is bound to one or more anion moieties.

The utility of the catalysts of our invention will now be illustrated by examples.

A series of runs which were carried out employing a variety of catalysts as shown in Table I. The runs were carried out as follows: the isobutane (6-7 gms) was dissolved in 25 ml benzene containing the catalyst, and air was added to the desired pressure. Oxidation was carried out at the designated temperature for the time listed in the tables. Gaseous and liquid products were analyzed by gas chromatography and mass spectrometry. Catalyst activity is expressed as "catalyst turnovers", i.e., moles of oxygen consumed/mole of catalyst. TBA is t-butyl alcohol. Selectivity is the moles of TBA per 100 moles of liquid product.

TABLE III

EFFECT OF RING HALOGENATION ON CATALYST ACTIVITY - ISOBUTANE OXIDATION

| CATALYST | NO. OF HALOGENS | CATALYST MMOLES | TIME HRS. | TEMP °C | CAT. TURNOVERS | SELECTIVITY |
|---|---|---|---|---|---|---|
| Fe(TPP)Cl | 0 | .025 | 6 | 80 | 0 | - |
| $Fe(TPPBr_4)Cl$ | 4 | .013 | 6 | 80 | 155 | N/A |
| $Fe(TPPCl_8)Cl$ | 8 | .019 | 6 | 80 | 260 | 89 |
| $Fe(TPPCL_8Br_4)Cl$ | 12 | .020 | 6 | 80 | 865 | 83 |
| $Fe(TPPF_{20})Cl$ | 20 | .016 | 6 | 80 | 2040 | 90 |
| $Fe(TPPF_{20}Br8)Cl$ | 28 | .013 | 6 | 80 | 3135 | 89 |
| $[Fe(TPP)]_2O$ | 0 | .007 | 6 | 80 | 0 | - |
| $(Fe(TPPBr_4)]_2O$ | 4 | .007 | 6 | 80 | 10 | N/A |
| $[Fe(TPPF_{20})]_2O$ | 20 | .007 | 6 | 80 | 1730 | 92 |
| $[Fe(TPPF_{20}Cl_8]_2O$ | 28 | .007 | 6 | 80 | 4180 | 80 |
| Mn(TPP)Cl | 0 | .013 | 6 | 80 | 0 | - |
| $Mn(TPPF_{20})Cl$ | 20 | .013 | 6 | 80 | trace | - |
| $Mn(TPPF_{20}Br_8)Cl$ | 28 | .013 | 6 | 80 | 422 | 88 |
| $Cr(TPP)N_3$ | 0 | .025 | 6 | 80 | 280 | 89 |
| $Cr(TPPF_{20})N_3$ | 20 | .016 | 6 | 80 | 450 | 97 |

The results shown in Table I demonstrate that perhalogenating the ligand of the metal coordination catalyst improves activity very remarkably compared with partial or unhalogenation.

## Claims

1. Catalyst composition useful for oxidizing alkanes having the formula

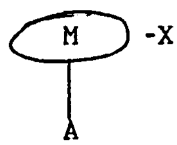

wherein 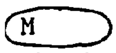 is a metal (perhalogenated porphyrin or perhalogenated phthalocyanine) coordination complex wherein the metal, (M), is iron, chromium, manganese, ruthenium, copper or combinations thereof, X is chloride, bromide, iodide, fluoride, or combinations thereof, and A is an anion in the case of phthalocyanine and is an anion or is absent in the case of porphyrin.

2. Catalyst according to Claim 1 wherein the metal is iron.

3. Catalyst according to Claim 1 wherein the metal is chromium.

4. Catalyst according to Claim 1 wherein the metal is manganese.

5. Catalyst according to any one of Claims 2, 3 or 4 wherein X is fluoride.

6. Catalyst according to any one of Claims 2, 3, or 4 wherein said anion is azide, halide, hydroxide or nitride.

7. Catalyst according to any one of Claims 1, 2, 3, or 4 wherein said halogenated coordination complex is perfluorotetra($C_1$-$C_4$)alkylporphyrin.

8. Catalyst according to any of Claims 1-4 wherein said coordination complex is phthalocyanine.

9. Catalyst according to any one of Claims 1-4 wherein said coordination complex is porphyrin.

10. Use of a catalyst according to any of claims 1 to 9 for oxidation by air or oxygen of an aliphatic hydrocarbon, which is optionally substituted by a non-interfering group.

11. A process for oxidising an aliphatic hydrocarbon, optionally substituted by a non-interfering group, which comprises contacting the hydrocarbon with air or oxygen in the presence of a catalyst according to any of claims 1 to 9.